(19) Europäisches Patentamt

European Patent Office

Office européen des brevets

(11) **EP 0 841 894 B1**

(12) **EUROPÄISCHE PATENTSCHRIFT**

(45) Veröffentlichungstag und Bekanntmachung des Hinweises auf die Patenterteilung:
**18.09.2002 Patentblatt 2002/38**

(51) Int Cl.[7]: **A61K 7/09**

(21) Anmeldenummer: **96943985.0**

(86) Internationale Anmeldenummer:
**PCT/EP96/05670**

(22) Anmeldetag: **17.12.1996**

(87) Internationale Veröffentlichungsnummer:
**WO 97/032563 (12.09.1997 Gazette 1997/39)**

(54) **MITTEL UND VERFAHREN ZUR DAUERHAFTEN HAARVERFORMUNG**

PERMANENT PROCESS AND AGENT

PROCEDE ET PRODUIT POUR PERMANENTE

(84) Benannte Vertragsstaaten:
**DE ES FR GB IT**

(30) Priorität: **06.03.1996 DE 19608581**

(43) Veröffentlichungstag der Anmeldung:
**20.05.1998 Patentblatt 1998/21**

(73) Patentinhaber: **Wella Aktiengesellschaft
64274 Darmstadt (DE)**

(72) Erfinder:
• **DANNECKER, Beate
D-64283 Darmstadt (DE)**
• **LANG, Günther
D-64354 Reinheim (DE)**
• **KELLER, Helmut
D-64287 Darmstadt (DE)**
• **BOHR, Ulrike
D-64372 Ober-Ramstadt (DE)**

(56) Entgegenhaltungen:
**EP-A- 0 624 472          DE-A- 4 326 974**

• **AGRICULTURAL AND BIOLOGICAL CHEMISTRY, Bd. 38, Nr. 11, November 1974, Seiten 2161-2165, XP000670895 Y. ODA ET AL : "The oxidation of d-1-thioglucose by cell-free extract from Aspergillus niger"**
• **SEIFEN-ÖLE-FETTE-WACHSE, Bd. 81, Nr. 3, 1955, Seiten 59-61, XP002029645 R. HEILLINGÖTTER: "Über die Wirksamkeit einer Mercaptoverbindung bei der Haarformung"**

**Beschreibung**

Die vorliegende Erfindung betrifft ein Mittel zur dauerhaften Haarverformung, welches als keratinreduzierenden Wirkstoff 1-Thio-β-D-glucose oder deren Salze, insbesondere das 1-Thio-β-D-glucose-Ammoniumsalz oder das Natriumsalz-Dihydrat enthält, sowie ein Verfahren zur dauerhaften Haarverformung unter Verwendung dieses Mittels.

Bekanntlich beruht die klassische Technik zur Durchführung einer dauerhaften Haarverformung auf zwei Behandlungsschritten: Im ersten Schritt werden die Cystin-Disulfid-Brücken des Haarkeratins durch Einwirken eines Mittels, welches einen reduzierenden Wirkstoff enthält (Verformungsmittel), gespalten. Sodann wird das Haar in die gewünschte Form gebracht. In einem zweiten Schritt werden Cystin-Disulfid-Bindungen unter Verwendung eines Fixiermittels, d.h. eines, einen oxidierenden Wirkstoff enthaltenden Mittels, wieder geschlossen.

Als klassisches Dauerwellreduktionsmittel wird, wie die Pionierarbeiten in den deutschen Patentschriften 948 186 und 972 424 zeigen, die Thioglykolsäure, z.B. als Ammonium- oder Monoethanolaminsalz, verwendet. Weitere übliche reduzierende Wirkstoffe sind anorganische Sulfite, 2-Mercapto-propionsäure (Thiomilchsäure), 3-Mercapto-propionsäure, bestimmte Mercaptocarbonsäureester, Cystein und Derivate dieser Verbindungen.

Alle diese Mittel weisen jedoch eine Reihe von Nachteilen auf. Alkalisch eingestellte Zubereitungen auf Basis der Thiocarbonsäuren zeigen trotz ausreichender Wirkung eine Haarschädigung, die sich beispielsweise in vermehrt auftretendem Haarbruch äußert. Vielfach belasten diese Mittel auch in unerwünschter Weise die Kopfhaut. Die Mercaptocarbonsäureester, welche eine Haarverformung auch im neutralen und leicht sauren Bereich ermöglichen, sind bezüglich ihrer Hautverträglichkeit sowie ihres Sensibilisierungsrisikos nicht zufriedenstellend.

Durch Verwendung von Thiomilchsäure ist man in der Lage, einige der erwähnten Probleme zu lösen. Allerdings zeichnet sich die Thiomilchsäure im Vergleich zur allgemein verwendeten Thioglykolsäure durch eine schwächere Umformung aus.

Die Anmelderin hat sich daher die Aufgabe gestellt, ein Haarverformungsmittel zur Verfügung zu stellen, welches das Haar bei dem haut- und haarschonenden pH-Bereich von 7,0 bis 8,5 in zufriedenstellender Weise dauerhaft verformt und keine physiologischen Nachteile aufweist.

Es wurde nun überraschend gefunden, daß sich die genannten Nachteile durch die Verwendung von 1-Thio-β-D-glucose oder dessen Salz , insbesondere 1-Thio-β-D-glucose-Natriumsalz-Dihydrat, vermeiden lassen und daß diese Verbindungen insbesondere im pH-Bereich zwischen 7,0 und 8,5 über ein stärkeres Umformungspotential als Thiomilchsäure verfügen.

Gegenstand der vorliegenden Erfindung ist daher ein Mittel zur dauerhaften Verformung der Haare auf der Basis eines keratinreduzierenden Wirkstoffes, welches dadurch gekennzeichnet ist, daß es als keratinreduzierenden Wirkstoff von 1-Thio-β-D-glucose oder deren Salz, insbesondere 1-Thio-β-D-glucose-Natriumsalz-Dihydrat, enthält. Als Salz ist insbesondere das Ammonium- oder Natriumsalz, aber auch jedes andere wasserlösliche, physiologisch verträgliche Salz mit organischen und anorganischen Basen geeignet.

Die 1-Thio-β-D-glucose oder ihr Salz soll in dem gebrauchsfertigen Mittel zur dauerhaften Haarverformung vorzugsweise in einer Menge von 3 bis 28 Gewichtsprozent, besonders bevorzugt in einer Menge von 5 bis 21 Gewichtsprozent, enthalten sein.

Das gebrauchsfertigen Haarverformungsmittel besitzt einen pH-Wert von 7 bis 8,5, vorzugsweise von 7,5 bis 8,5, wobei die Alkalisierung in der Regel, neben Ammoniak, durch Zusatz von Ammoniumhydrogencarbonat erzielt wird.

Die 1-Thio-β-D-glucose oder deren Salz ist in dem Mittel bevorzugt als alleiniger keratinreduzierender Wirkstoff enthalten. Sie kann jedoch auch in Kombination mit anderen keratinreduzierenden Wirkstoffen - wie zum Beispiel Thioglykolsäure, Thiomilchsäure, 2-Hydroxy-3-mercapto-propionsäure, Cysteamin und Cysteaminderivaten oder Cystein und Cysteinderivaten, z. B. Cystein-(2-hydroxyethyl)-ester oder L-Cystein-glycerinester-eingesetzt werden.

Das Verformungsmittel kann als Ein-, Zwei- oder Dreikomponenten-Präparat konfektioniert sein, wobei das Mittel sowohl in Form einer wäßrigen Lösung oder einer Emulsion als auch in verdickter Form auf wäßriger Basis, insbesondere als Creme, Gel oder Paste, vorliegen kann.

So ist das erfindungsgemäße Mittel zum Beispiel durch Vermischen zweier Komponenten erhältlich, von denen die erste Komponente mindestens ein Alkalisierungsmittel, beispielsweise ein Alkalicarbonat, Ammoniumcarbonat, Alkalihydrogencarbonat oder Ammoniumhydrogencarbonat, und 1-Thio-β-D-glucose oder dessen Salz enthält und die zweite Komponente mindestens eine der nachstehend genannten kosmetischen Zusatzstoffe und Wasser enthält.

Ebenfalls ist es möglich, das erfindungsgemäße Mittel in Form eines Dreikomponenten-Präparates abzupacken, wobei eine Komponente einige der nachfolgend genannten kosmetischen Zusatzstoffe sowie Wasser enthält, eine zweite, wasserfreie Komponente die 1-Thio-β-D-glucose oder deren Salz enthält und die dritte Komponente weitere Zusatzstoffe, wie z. B. Parfümöle, Lösungsvermittler und Pflegestoffe, in wäßriger Lösung oder in wasserfreier Form enthält.

Bei allen Ausführungsformen des erfindungsgemäßen Mittels können die kosmetischen Zusatzstoffe sowohl in der wäßrigen als auch in der/den nichtwäßrigen Komponente(n) enthalten sein.

Selbstverständlich kann das Verformungsmittel alle für derartige Mittel üblichen und bekannten Zusatzstoffe, zum Beispiel Verdickungsmittel, wie beispielsweise, Bentonit, Fettsäuren höherer Alkohole, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; Alkohole, wie zum Beispiel Ethanol, Propanol, Isopropanol und Glycerin; Zucker wie z. B. D-Glucose; Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure und Betain, enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0,01 bis 1 Gewichtsprozent, die Puffersubstanzen in einer Menge von insgesamt 0,1 bis 10 Gewichtsprozent, Zucker, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 2 bis 30 Gewichtsprozent sowie zur Vermeidung einer Überkrausung der Haare Dithioverbindungen, beispielsweise Dithiodiglykolsäure, Dithiomilchsäure oder deren Salze, zugesetzt werden.

Durch Variation des pH-Wertes kann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte und gleichmäßige Umformung vom Haaransatz bis zur Haarspitze, ohne allergische oder sensibilisierende Reaktionen hervorzurufen.

Die vorliegende Erfindung betrifft weiterhin ein Verfahren zur dauerhaften Haarverformung, bei dem man die Haare bevor und/oder nachdem man sie in der gewünschte Form festhält, mit einem Verformungsmittel behandelt, mit Wasser spült, sodann oxidativ behandelt, mit Wasser spült, gegebenenfalls zur Wasserwelle legt und sodann trocknet, welches dadurch gekennzeichnet ist, daß als Verformungsmittel die vorstehend beschriebenen erfindungsgemäßen Mittel verwendet werden.

Bei einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung im aufgewickelten oder abgewickelten Zustand kann jedes beliebige, für eine derartige Behandlung geeignetes Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gew.% in dem Nachbehandlungsmittel enthalten sein.

Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen den Gegenstand der Erfindung näher erläutern, ohne jedoch den Gegenstand auf diese Beispiele zu beschränken.

**Beispiele**

**Beispiel 1: Vergleich der Wellwirksamkeiten**

Die Wellwirksamkeit von 1-Thio-β-D-glucose-Natriumsalz-Dihydrat wurde unter Verwendung von Glycerinmonothioglykolat als Vergleichssubstanz mit Hilfe von Wellösungen bei pH = 7, 8 und 9 bestimmt. Hierzu wurden 16,5 Zentimeter lange, vorgebleichte und damit geschädigte Zählhaarsträhnen (bestehend aus ca. 100 Haaren) aus mitteleuropäischem Haar naß auf genormte Spiralwickler (Innendurchmesser: 3 Millimeter) aufgewickelt und nach dem Konditionieren im Klimaraum (Temperatur: 20°C; Luftfeuchte: 65%) mit einer 87 mmol/100g enthaltenden, auf den jeweiligen pH-Wert eingestellten Lösung der Reduktionsmittel behandelt. Die Auftragemenge an Wellflüssigkeit wurde über das Verhältnis 1 : 1,2 errechnet (1g Haar : 1,2 ml Wellflüssigkeit). Als Einwirkungszeit wurden 20 Minuten gewählt; die Einwirkungstemperatur betrug 50 Grad Celsius. Anschließend wurden die Haare mit einer peroxidhaltigen Fixierung fixiert, getrocknet und nach dem Abwickeln vier Stunden in Wasser (Wasserbadtmepratur: 40°C) ausgehängt.

Die Wellstabilität errechnet sich nach folgender Formel:

$$\text{Wellstabilität in \%} = \frac{I_o - I_t}{I_o - I_t} \times 100$$

| $I_o =$ | Gesamtlänge der nicht umgeformten, gestreckten Strähne (16,5 Zentimeter) |
|---|---|
| $I_t =$ | Länge der abgewickelten, ausgehängten Strähne nach 240 Minuten |
| $I_1 =$ | Länge der umgeformten, aufgewickelten Strähne |
| | bei einem Wickelinnendurchmesser von 3 mm beträgt diese: $I_1 = 35$ Millimeter |

Als Standard wurden Strähnchen mit einer entsprechend auf pH 9 eingestellten Glycerinmonothioglykolat-Lösung behandelt. Die in Tabelle I angegebenen normierten Wellstabilitäten beziehen sich auf diese Standardlösung (pH = 9), deren Wellstabilität auf 100 Prozent gesetzt wurde.

Tabelle I:

| Normierte Wellstabilitäten (WSN) in %. | | |
|---|---|---|
| **pH der Wellösung** | **WSN von Thiomilchsäure** | **WSN von 1-Thio-β-D-glucose-Natriumsalz-Dihydrat** |
| 7 | 57% | 63% |
| 8 | 50% | 63% |
| 9 | 70% | 59% |

Tabelle I zeigt, daß die Wellwirksamkeit von 1-Thio-β-D-glucose-Natrlumsalz-Dihydrat bei pH 7 und pH 8 höher ist als bei Thiomilchsäure.

**Beispiel 2: Dauerverformungsmittel für gefärbtes Haar**

| 12,0 g | 1-Thio-β-D-glucose-Natriumsalz-Dihydrat |
|---|---|
| 0,4 g | Ammoniak (25%-ige wäßrige Lösung) |
| 2,0 g | Ammoniumhydrogencarbonat |
| 2,0 g | Isopropanol |
| 1,0 g | Isooctylphenol, oxethyliert mit 10 Mol Ethylenoxid |
| 1,0 g | Polydimethyldiallylammoniumchlorid |
| 0,3 g | Parfümöl |
| 0,1 g | Vinylpyrrolidon/Styrol-Mischpolymerisat (Antara® 430 der GAF Corp.; New York/USA) |
| 81,2g | Wasser |
| 100,0 g | |

Der pH-Wert dieses Mittels liegt im Bereich von 7,0 - 7,5.

Durch Farbbehandlungen vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 10 Minuten lang unter einer Trockenhaube bei einer Temperatur von 45 Grad Celsius erwärmt. Anschließend wird die Abdeckung entfernt, das Haar mit Wasser gespült und mit 100 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und sodann getrocknet.

Als Ergebnis dieser Behandlung wird eine gleichmäßige, elastische und dauerhafte Verformung der Haare erhalten.

**Beispiel 3: Dauerverformungsmittel für normales Haar**

| 17,5 | 1-Thio-ß-D-glucose-Natiumsalz-Dihydrat |
|---|---|
| 8,9 g | Ammoniak (25%-ige wäßrige Lösung) |
| 5,0 g | Ammoniumhydrogencarbonat |
| 2,0 g | D-Glucose |
| 2,4 g | Ammoniak |
| 1,5 g | Isooctylphenol, oxethyliert mit 10 Mol Ethylenoxid |
| 0,5 g | Polydimethyldiallylammoniumchlorid |
| 0,5 g | Parfümöl |
| 0,1 g | Vinylpyrrolidon/Styrol-Mischpolymerisat (Antara® 430 der GAF Corp.; New York/USA) |
| 61,6g | Wasser |
| 100,0 g | |

Der pH-Wert dieses Mittels liegt im Bereich von 8,0 - 8,5.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 - 25 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet.

Das so behandelte Haar besitzt eine gleichmäßige und lebhafte Krause.

**Patentansprüche**

1. Mittel zur dauerhaften Verformung von Haaren auf der Basis eines keratinreduzierenden Wirkstoffes, **dadurch gekennzeichnet, daß** es als keratinreduzierenden Wirkstoff 1-Thio-β-D-glucose oder deren Salz enthält.

2. Mittel nach Anspruch 1, **dadurch gekennzeichnet, daß** es als alleinigen keratinreduzierenden Wirkstoff 1-Thio-β-D-glucose oder deren Salz enthält.

3. Mittel nach einem der Ansprüche 1 bis 2, **dadurch gekennzeichnet, daß** die 1-Thio-β-D-glucose oder deren Salz in dem gebrauchsfertigen Mittel in einer Menge von 3 bis 28 Gewichtsprozent enthalten ist.

4. Mittel nach einem der Ansprüche 1 bis 3, **dadurch gekennzeichnet, daß** der pH-Wert des gebrauchsfertigen Mittels 7,0 bis 8,5 beträgt.

5. Mittel nach einem der Ansprüche 1 bis 4, **dadurch gekennzeichnet, daß** es vor der Anwendung durch Vermischen von zwei oder drei Komponenten erhalten wird.

6. Verfahren zur dauerhaften Verformung von Haaren, bei dem man das Haar bevor und/oder nachdem man es in der gewünschten Form festhält, mit einem Verformungsmittel behandelt, mit Wasser spült, oxidativ nachbehandelt, erneut mit Wasser spült, gegebenenfalls zur Wasserwelle legt und sodann trocknet, **dadurch gekennzeichnet, daß** man als Verformungsmittel ein Mittel nach einem der Ansprüche 1 bis 5 verwendet.

7. Verfahren nach Anspruch 6, **dadurch gekennzeichnet, daß** man das Verformungsmittel 5 bis 30 Minuten lang einwirken läßt.

8. Verfahren nach Anspruch 6, **dadurch gekennzeichnet, daß** man das Verformungsmittel unter Anwendung von Wärme 5 bis 20 Minuten lang einwirken läßt.

9. Verfahren nach einem der Ansprüche 6 bis 8, **dadurch gekennzeichnet, daß** man das Verformungsmittel in einer Menge von 60 bis 120 Gramm anwendet.

**Claims**

1. Agent for the permanent shaping of hair based on a keratin-reducing active ingredient, **characterized in that** it comprises, as keratin-reducing active ingredient, 1-thio-$\beta$-D-glucose or salt thereof.

2. Agent according to Claim 1, **characterized in that** it comprises, as the sole keratin-reducing active ingredient, 1-thio-$\beta$-D-glucose or salt thereof.

3. Agent according to either Claim 1 or 2, **characterized in that** the 1-thio-$\beta$-D-glucose or salt thereof is present in the ready-to-use agent in an amount of from 3 to 28% by weight.

4. Agent according to any of Claims 1 to 3, **characterized in that** the pH of the ready-to-use agent is 7.0 to 8.5.

5. Agent according to any of Claims 1 to 4, **characterized in that** it is obtained by mixing two or three components prior to application.

6. Method for the permanent shaping of hair in which the hair, before and/or after being held in the desired shape, is treated with a shaping agent, rinsed with water, oxidatively after-treated, rinsed with water again, optionally arranged in a water-wave and then dried, **characterized in that** the shaping agent used is an agent according to one of Claims 1 to 5.

7. Method according to Claim 6, **characterized in that** the shaping agent is left to act for 5 to 30 minutes.

8. Method according to Claim 6, **characterized in that** the shaping agent is left to act, with the application of heat, for 5 to 20 minutes.

9. Method according to one of Claims 6 to 8, **characterized in that** the shaping agent is applied in an amount of from 60 to 120 grams.

**Revendications**

1. Composition pour l'ondulation permanente des cheveux, à base d'un ingrédient actif réducteur de kératine, **caractérisée en ce qu'**elle comprend du 1-thio-$\beta$-D-glucose ou son sel en temps qu'ingrédient actif réducteur de kératine.

2. Composition selon la revendication 1, **caractérisée en ce qu'**elle comprend du 1-thio-$\beta$-D-glucose ou son sel en tant qu'unique ingrédient actif réducteur de kératine.

3. Composition selon l'une quelconque des revendications 1 à 2, **caractérisée en ce que** le 1-thio-$\beta$-D-glucose ou son sel est présent dans la composition prête à l'emploi en une quantité de 3 à 28 % en poids.

4. Composition selon l'une quelconque des revendications 1 à 3, **caractérisée en ce que** le pH de la composition prête à l'emploi est de 7,0 à 8,5.

5. Composition selon l'une quelconque des revendications 1 à 4, **caractérisée en ce qu'**elle est obtenue en mélangeant deux ou trois composants avant l'application.

6. Procédé d'ondulation permanente des cheveux, dans lequel on traite les cheveux par une composition ondulante avant et/ou après les avoir fixés sous la forme souhaitée, on les rince à l'eau, on les soumet à un post-traitement oxydatif, on les rince de nouveau à l'eau, on les soumet éventuellement à une mise en plis, puis on les sèche, **caractérisé en ce que** l'on utilise une composition selon l'une quelconque des revendications 1 à 5 en tant que composition ondulante.

7. Procédé selon la revendication 6, **caractérisé en ce que** l'on laisse la composition ondulante agir pendant 5 à 30 minutes.

8. Procédé selon la revendication 6, **caractérisé en ce que** l'on laisse la composition ondulante agir pendant 5 à 20 minutes en appliquant de la chaleur.

9. Procédé selon l'une quelconque des revendications 6 à 8, **caractérisé en ce que** la composition ondulante est appliquée en une quantité de 60 à 120 grammes.